# EUROPÄISCHE PATENTSCHRIFT

(11) **EP 3 010 941 B1**
(45) Veröffentlichungstag und Bekanntmachung des Hinweises auf die Patenterteilung: **28.11.2018**
(21) Anmeldenummer: 14747794.7
(22) Anmeldetag: 13.06.2014
(51) Int. Cl.: C08B 37/00, D01D 5/06, D01F 9/00

(54) **VERFAHREN ZUR HERSTELLUNG VON SACCHARIDFASER**
METHOD FOR PRODUCING SACCHARIDE FIBRES
PROCÉDÉ DE PRODUCTION DE FIBRES SACCHARIDES

(30) Priorität: 18.06.2013 AT 4902013
(43) Veröffentlichungstag der Anmeldung: 27.04.2016
(73) Patentinhaber: Lenzing AG, 4860 Lenzing (AT)
(72) Erfinder: KRAFT, Gregor, A-4860 Lenzing (AT); KRONER, Gert, 4863 Seewalchen (AT); RÖDER, Thomas, A-4840 Vöcklabruck (AT); FIRGO, Heinrich, 4840 Vöcklabruck (AT)
(74) Vertreter: Hanemann, Otto
(86) Internationale Anmeldenummer: PCT/AT2014/000122
(87) Internationale Veröffentlichungsnummer: WO 2014/201481

(56) Entgegenhaltungen:
- WO-A1-2013/036968
- WO-A1-2013/052730
- US-A- 4 306 059
- US-A1- 2013 161 861
- Akira Misaki: Foods Food Ingredients J. Jpn, 1. Januar 2004 (2004-01-01), XP055144241, Gefunden im Internet: URL:http://www.ffcr.or.jp/zaidan/ffcrhome. nsf/7bd44c20b0dc562649256502001b65e9/a574b e4bca4c288149256e82000f39bb/$FILE/209(4)3. pdf [gefunden am 2014-10-02]
- ZHANG P ET AL: "Effects of urea and sodium hydroxide on the molecular weight and conformation of alpha-(1->3)-d-glucan from Lentinus edodes in aqueous solution", CARBOHYDRATE RESEARCH, PERGAMON, GB, Bd. 327, Nr. 4, 7. August 2000 (2000-08-07), Seiten 431-438, XP004213354, ISSN: 0008-6215, DOI: 10.1016/S0008-6215(00)00077-X

## Beschreibung

Die vorliegende Erfindung betrifft ein Direktlöseverfahren zur Herstellung von Polysaccharidfasern, die als faserbildende Substanz α(1→3)-Glucan enthalten, mit Natronlauge als Lösungsmittel.

### Stand der Technik

Polysaccharide spielen als Materialien, die aus nachwachsenden Rohstoffen gewonnen werden können, eine immer größere Rolle. Eines der am häufigsten vorkommenden Polysaccharide ist die Cellulose. Baumwollfasern, die fast ausschließlich aus Cellulose bestehen, sind ein Beispiel für die Wichtigkeit von Polysacchariden. Aber auch aus anderen cellulosischen Rohstoffen gewonnene Materialien wie z. B. cellulosische Kunstfasern gewinnen immer mehr an Bedeutung.

Die Gattungsnamen "Viscose-Fasern" und "Modal-Fasern" wurden von der BISFA (Bureau for the International Standardization of Man-made Fibers) Cellulosefasern zugeteilt, die durch chemische Derivatisierung von Cellulose mithilfe von Natronlauge und Schwefelkohlenstoff (CS2) hergestellt werden. Der Name "Modalfaser" ist ein generischer Begriff, der gemäß der Definition der BISFA für eine Cellulosefaser mit einer definierten hohen Nassfestigkeit und einem ebenfalls definierten hohen Nassmodul (d.h. die Kraft, welche benötigt wird, um die Faser in nassem Zustand um 5% zu dehnen) steht.

Bis heute hat sich jedoch nur ein Verfahren zur großtechnischen Herstellung von Fasern der Gattungen Viscose und Modal durchgesetzt und zwar das Viskose-Verfahren und Abwandlungen davon.

Wie dieses Verfahren ausgeführt wird, ist dem Fachmann grundsätzlich seit längerem aus vielen Patentschriften und sonstigen Publikationen bekannt. Ein Verfahren zur Herstellung von Modalfasern ist beispielsweise aus der AT 287.905 B bekannt.

Der große Nachteil aller Viskoseverfahren ist der Einsatz von CS2, das mit großem Aufwand zurück gewonnen werden muss.

Der Gattungsname "Lyocell-Fasern" wurde von der BISFA Cellulosefasern zugeteilt, die aus Lösungen in einem organischen Lösungsmittel ohne Bildung eines Derivats hergestellt werden.

Bis heute hat sich jedoch nur ein Verfahren zur großtechnischen Herstellung von Fasern der Gattung Lyocell durchgesetzt und zwar das Aminoxid-Verfahren. Bei diesem Verfahren wird als Lösungsmittel ein tertiäres Aminoxid verwendet, bevorzugt N-Methylmorpholin-N-oxid (NMMO).

Tertiäre Aminoxide sind schon seit langem als alternative Lösungsmittel für Cellulose bekannt. Aus der US 2,179,181 ist beispielsweise bekannt, dass tertiäre Aminoxide Zellstoff ohne Derivatisierung zu lösen vermögen und dass aus diesen Lösungen cellulosische Formkörper wie z.B. Fasern hergestellt werden können. In der US 3,447,939 werden cyclische Aminoxide als Lösungsmittel für Cellulose beschrieben.

Wie dieses Verfahren ausgeführt wird, ist dem Fachmann grundsätzlich seit längerem aus vielen Patentschriften und sonstigen Publikationen bekannt. So beschreibt unter anderem die EP 356 419 B1 die Lösungsherstellung und die EP 584 318 B1 das Verspinnen solcher Lösungen von Cellulose in wasserhaltigen tertiären Aminoxiden.

Das Lyocellverfahren ist als Direktlöseverfahren aus ökologischer Sicht wesentlich unbedenklicher als die Viskoseverfahren, hat aber durch die wirtschaftliche Notwendigkeit einer nahezu vollständigen Kreislaufschließung verfahrenstechnische Nachteile, da sich Substanzen in den Kreisläufen anreichern können.

Die US 7,000,000 beschreibt Fasern, die durch Verspinnen einer Lösung von Polysacchariden, die im Wesentlichen aus Hexose-Wiederholungseinheiten bestehen, die über α(1→3)-glycosidische Bindungen verknüpft sind, erhalten werden. Diese Polysaccharide können hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)). "Im Wesentlichen" bedeutet in diesem Zusammenhang, dass innerhalb der Polysaccharidketten vereinzelt Fehlstellen auftreten können, an denen andere Bindungskonfigurationen auftreten. Diese Polysaccharide sollen für die Zwecke der vorliegenden Erfindung als "α(1→3)-Glucan" bezeichnet werden.

Die US 7,000,000 offenbart zunächst Möglichkeiten zur enzymatischen Herstellung von α(1→3)-Glucan aus Monosacchariden. Auf diese Weise können relativ kurzkettige Polysaccharide ohne Verlust an Monomerbausteinen hergestellt werden, da die Polymerketten aus den Monomerbausteinen aufgebaut werden. Im Gegensatz zur Herstellung kurzkettiger Cellulosemoleküle ist die Herstellung von (α(1→3)-Glucan umso preisgünstiger, je kürzer die Polymerketten sind, da dann nur eine geringe Verweilzeit in den Reaktoren notwendig ist.

Gemäß der US 7,000,000 soll das α(1→3)-Glucan derivatisiert, bevorzugt acetyliert, werden. Das Lösungsmittel ist bevorzugt eine organische Säure, eine organische Halogenverbindung, ein fluorierter Alkohol oder eine Mischung aus solchen Komponenten. Diese Lösungsmittel sind teuer und aufwendig zu regenerieren.

Die WO 2013/052730 A1 offenbart Fasern mit α(1→3)-Glucan als faserbildende Substanz, die nach dem sogenannten Aminoxid- oder LyocellVerfahren mit NMMO als Lösungsmittel durch Verspinnen hergestellt wurden. Das Aminoxid-Verfahren erfordert eine aufwendige Kreislaufführung des Lösungsmittels, das zu einem sehr hohen Prozentsatz zurückgewonnen werden muß. Die gemäß der WO 2013/052730 A1, insbesondere in Beispielen 1 bis 3, hergestellten Fasern weisen jedoch nur eine geringe Festigkeit von 0,8 Gramm pro Denier auf.

Untersuchungen haben aber auch gezeigt, dass α(1→3)-Glucane in verdünnter Natronlauge löslich sind.

### Aufgabe

Die Aufgabe bestand gegenüber diesem Stand der Technik daher darin, ein alternatives Direktlöseverfahren zur Herstellung von Polysaccharid-Fasern zur Verfügung zu stellen, das ohne das im Viskoseverfahren notwendige CS2 und ohne die aufwendige Kreislaufschließung eines Lyocellverfahrens auskommt.

### Beschreibung der Erfindung

Die Lösung der oben beschriebenen Aufgabe besteht in einem neuen Direktlöseverfahren zur Herstellung einer Polysaccharid-Faser, deren faserbildende Substanz α(1→3)-Glucan ist, wobei das Direktlöseverfahren auf Natronlauge basiert.

Gegenstand der vorliegenden Erfindung ist daher zum einen ein Verfahren zur Herstellung einer Polysaccharid-Faser, deren faserbildende Substanz α(1→3)-Glucan ist, wobei das Verfahren ein Direktlöseverfahren ist und das Lösemittel Natronlauge ist.

Überraschenderweise wurde festgestellt, dass Standardspinnbäder, wie sie im Viskoseverfahren eingesetzt werden (diese enthalten etwa 100g/l H2SO4 und ca. 250 g/l Na2SO4), sehr schlechte Resultate liefern, aber zwei andere, sehr unterschiedliche Spinnbadzusammensetzungen deutlich bessere Ergebnisse liefern.
1. Hochsäurespinnen: Es wurde festgestellt, dass sich bei Erhöhung der Schwefelsäurekonzentration im Spinnbad die Fadenbildung und Verstreckbarkeit des Regeneratfadens deutlich verbessert. Der untersuchte Bereich mit gutem Spinnverhalten erstreckt sich von 200 bis 500 Gramm Schwefelsäure je Liter Spinnbad.
2. Niedrigstsäurespinnen: der zweite Spinnbereich, der eine deutlich bessere Spinnsicherheit zeigte, liegt bei sehr niedrigen Säurekonzentrationen unterhalb 60 Gramm pro Liter Spinnbad, bevorzugt 20 - 60 g/l. Gute Ergebnisse wurden auch mit einem Zweibad-System erreicht, bei dem das erste Bad sehr hohe Salzgehalte und eine sehr niedrige Säurekonzentration aufweist, wodurch der Spinnfaden zunächst nur koaguliert und erst im zweiten sauren Regenerierbad regeneriert wird.

In einer bevorzugten Ausführungsform des erfindungsgemäßen Verfahrens beträgt daher die H2SO4-Konzentration im Spinnbad zwischen 200 und 500 g/l.

In einer zweiten bevorzugten Ausführungsform des erfindungsgemäßen Verfahrens beträgt daher die H2SO4-Konzentration im Spinnbad zwischen 20 und 60 g/l.

In einer bevorzugten Ausführungsform des erfindungsgemäßen Verfahrens wird die ausgesponnene Faser anschließend in einem sauren Regenerierbad verstreckt.

Die NaOH-Konzentration in der Spinnlösung soll erfindungsgemäß zwischen 4,0 und 5,5 Gew.-%, bezogen auf die Gesamtmenge der Spinnlösung betragen. Außerhalb dieses Bereichs ist die Löslichkeit des Glucans nicht ausreichend gegeben.

Der Begriff "Faser" sowohl Stapelfasern mit definierter Schnittlänge als auch Endlosfilamente umfassen. Sämtliche im Folgenden beschriebenen Prinzipien gelten grundsätzlich sowohl für Stapelfasern als auch für Endlosfilamente.

Der Einzelfasertiter der Fasern kann zwischen 0,1 und 10 dtex betragen. Bevorzugt ist er zwischen 0,5 und 6,5 dtex und besonders bevorzugt zwischen 0,9 und 6,0 dtex. Im Falle von Stapelfasern beträgt die Schnittlänge üblicherweise zwischen 0,5 und 120 mm, bevorzugt zwischen 20 und 70 mm und besonders bevorzugt zwischen 35 und 60 mm. Im Falle von Endlosfilamenten beträgt die Anzahl der Einzelfilamente im Filamentgarn zwischen 50 und 10.000, bevorzugt zwischen 50 und 3.000.

Das α(1→3)-Glucan kann hergestellt werden, indem eine wässrige Lösung von Saccharose mit Glucosyltransferase (GtfJ), isoliert aus Streptococcus salivarius, in Kontakt gebracht wird (Simpson et al. Microbiology, vol 41, pp 1451-1460 (1995)).

In einer bevorzugten Ausführungsform des erfindungsgemäßen Verfahrens besteht das α(1→3)-Glucan zu mindestens 90 % aus Hexose-Einheiten und mindestens 50 % der Hexose-Einheiten sind durch α(1→3)-glycosidische Bindungen verknüpft.

Das Verfahren zur Herstellung der erfindungsgemäßen Faser besteht aus folgenden Schritten:
1. Herstellung einer α(1→3)-Glucan-Lösung in verdünnter Natronlauge
2. Ausspinnen der α(1→3)-Glucan-haltigen Spinnlösung durch eine Düse in ein schwefelsaures Spinnbad, Verstreckung der Fasern in einem sauren Regenerierbad und Nachbehandlung.

Die Konzentration der faserbildenden Substanz in der Spinnlösung kann zwischen 4 und 18 Gew.-% betragen, bevorzugt sind 4,5 bis 12 Gew.-%.

Der Polymerisationsgrad des im erfindungsgemäßen Verfahren eingesetzten α(1→3) Glucans, ausgedrückt als Gewichtsmittel DP_{w}, kann zwischen 200 und 2000 liegen; bevorzugt sind Werte zwischen 500 und 1000.

In einer bevorzugten Ausführungsform besteht das α(1→3)-Glucan der erfindungsgemäßen Polysaccharid-Faser zu mindestens 90 % aus Hexose-Einheiten und mindestens 50 % der Hexose-Einheiten sind durch α(1→3)-glycosidische Bindungen verknüpft.

Die erhaltenen Fasern können zur Herstellung von textilen Erzeugnissen wie Garnen, Geweben, Gestricken oder Gewirken sowie auch von verschiedensten trocken- und nass gelegten Papieren, Vliesstoffen, Hygieneartikeln wie Tampons, Slipeinlagen und Windeln und sonstigen Vliesstoffen verwendet werden, insbesondere absorbierenden Nonwovens-Produkten.

Im Folgenden wird die Erfindung anhand von Beispielen beschrieben. Die Erfindung ist jedoch ausdrücklich nicht auf diese Beispiele beschränkt, sondern umfasst auch alle anderen Ausführungsformen, die auf dem gleichen erfinderischen Konzept beruhen, wie es in den Ansprüchen spezifiziert wird.

### Beispiele

Der Polymerisationsgrad der α(1→3)-Glucane wurde mittels GPC in DMAc/LiCl ermittelt. Im Folgenden wird stets das Gewichtsmittel des Polymerisationsgrades (DP_{w}) angegeben.

### Beispiel 1:

Eine wässrige Glucanlösung, enthaltend 9% α(1→3)-Glucan mit einem DP_{w} von 800 sowie 4,5 Gew.-% NaOH wurde auf 3 °C abgekühlt, filtriert und entlüftet. Die Lösung wurde mittels einer Spinndüse in ein Spinnbad mit 35 °C, enthaltend 300 g/l Schwefelsäure und 50 g/l Natriumsulfat gesponnen. Die Spinndüse hatte 53 Löcher mit 50µm Durchmesser. Zur Erzielung einer entsprechenden Faserfestigkeit erfolgte eine Verstreckung im Regenerierbad (97°C, 25 g/l H2S04). Die Abzugsgeschwindigkeit betrug 30 m/min.

Die Eigenschaften der erhaltenen Fasern sind in der Tabelle 1 angegeben:

### Beispiel 2:

Eine wässrige Glucanlösung, enthaltend 9% α(1→3)-Glucan mit einem DP_{w} von 1000 sowie 4,8 Gew.-% NaOH wurde auf 0 °C abgekühlt, filtriert und entlüftet. Die Lösung wurde mittels einer Spinndüse in ein Spinnbad mit 20 °C, enthaltend 35 g/l Schwefelsäure, 280 g/l Natriumsulfat und 45 g/l Zinksulfat, gesponnen. Die Spinndüse hatte 53 Löcher mit 40 µm Durchmesser. Zur Erzielung einer entsprechenden Faserfestigkeit erfolgte eine Verstreckung im Regenerierbad (92°C, 55 g/l H2S04). Die Abzugsgeschwindigkeit betrug 25 m/min. Die Eigenschaften der erhaltenen Fasern sind in der Tabelle 1 angegeben:

**Tabelle 1**

| Beispiel | Titer dtex | FFk cN/tex | FDk % |
|---|---|---|---|
| Bsp 1 | 1,7 | 15,3 | 11,1 |
| Bsp 2 | 1,7 | 19,1 | 9,2 |

| | | | |
|---|---|---|---|
| FFk Faserfestigkeit konditioniert FDk Faserdehnung konditioniert | | | |

## Patentansprüche

1. Verfahren zur Herstellung einer Polysaccharid-Faser, deren faserbildende Substanz α(1→3)-Glucan ist, **dadurch gekennzeichnet, dass** das Verfahren ein Direktlöseverfahren ist und das Lösemittel Natronlauge ist.

2. Verfahren nach Anspruch 1, wobei die NaOH-Konzentration in der Spinnlösung zwischen 4,0 und 5,5 Gew.-%, bezogen auf die Gesamtmenge der Spinnlösung, beträgt.

3. Verfahren nach Anspruch 1, wobei die H2SO4-Konzentration im Spinnbad zwischen 200 und 500 g/l beträgt.

4. Verfahren nach Anspruch 1, wobei die H2SO4-Konzentration im Spinnbad zwischen 20 und 60 g/l beträgt.

5. Verfahren nach Anspruch 1, wobei die ausgesponnene Faser anschließend in einem sauren Regenerierbad verstreckt wird.

6. Verfahren nach Anspruch 1, wobei das α(1→3)-Glucan zu mindestens 90 % aus Hexose-Einheiten besteht und mindestens 50 % der Hexose-Einheiten durch α(1→3)-glycosidische Bindungen verknüpft sind.

7. Verfahren gemäß den vorhergehenden Ansprüchen, wobei die Faser eine Stapelfaser oder ein Endlosfilament ist.

## Claims

1. A method for the production of a polysaccharide fiber whose fiber-forming substance is α(1→3)-glucan, **characterized in that** the method is a direct dissolving process and that the solvent is aqueous sodium hydroxide solution.

2. The method as claimed in claim 1, wherein the NaOH concentration in the spinning solution is between 4.0 and 5.5% by weight related to the total quantity of the spinning solution.

3. The method as claimed in claim 1, wherein the H₂SO₄ concentration in the spin bath is between 200 and 500 g/l.

4. The method as claimed in claim 1, wherein the H₂SO₄ concentration in the spin bath is between 20 and 60 g/l.

5. The method as claimed in claim 1, wherein the spun fiber is subsequently stretched in an acid regeneration bath.

6. The method as claimed in claim 1, wherein at least 90% of the α(1→3)-glucan are hexose units and at least 50% of the hexose units are linked via α(1→3)-glycosidic bonds.

7. The method as claimed in any of the preceding claims, wherein the fiber is a staple fiber or a continuous filament.

## Revendications

1. Procédé pour la production d'une fibre polysaccharidique dont la substance formant les fibres est l'α(1→3)-glucane, **caractérisé en ce que** ledit procédé est un procédé de dissolution directe et que le solvant est la soude caustique.

2. Procédé selon la revendication 1, la concentration en NaOH dans la solution à filer se situant entre 4,0 et 5,5 % en poids par rapport à la quantité totale de la solution à filer.

3. Procédé selon la revendication 1, la concentration en H2SO4 dans le bain de filage se situant entre 200 et 500 g/l.

4. Procédé selon la revendication 1, la concentration en H2SO4 dans le bain de filage se situant entre 20 et 60 g/l.

5. Procédé selon la revendication 1, la fibre filée étant ensuite étirée dans un bain acide de régénération.

6. Procédé selon la revendication 1, l'α(1→3)-glucane étant composé d'au moins 90 % d'unités d'hexose, au moins 50 % des unités d'hexose étant reliées par des liaisons glycosidiques α(1→3).

7. Procédé selon les revendications précédentes, ladite fibre étant une fibre discontinue ou un filament continu.
